Europäisches Patentamt

European Patent Office

Office européen des brevets

(11) Publication number: **0 224 599**
**A1**

# (12) EUROPEAN PATENT APPLICATION

(21) Application number: **85114149.9**

(22) Date of filing: **06.11.85**

(51) Int. Cl.⁴: **A61K 7/22**

Amended claims in accordance with Rule 86 (2) EPC.

(43) Date of publication of application:
**10.06.87 Bulletin 87/24**

(84) Designated Contracting States:
**AT BE CH DE FR GB IT LI LU NL SE**

(71) Applicant: **NATIONAL PATENT DEVELOPMENT CORPORATION**
**375 Park Avenue - Suite 2201**
**New York, NY 10152(US)**

(72) Inventor: **Moro, Daniel G.**
**7 Carriage Court**
**Randolph, New Jersey 07869(US)**

(74) Representative: **Schmidt-Evers, Jürgen,**
**Dipl.-Ing. et al**
**Patentanwälte Dipl.-Ing. H. Mitscherlich**
**Dipl.-Ing. K. Gunschmann Dipl.-Ing.**
**Dr.rer.nat. W. Körber Dipl.-Ing. J.**
**Schmidt-Evers Dipl.-Ing. W. Melzer**
**Steinsdorfstrasse 10**
**D-8000 München 22(DE)**

(54) Chemical solution useful in the treatment of teeth.

(57) There is provided a chemical solution useful in the removal of carious lesions or plaque, said solution being prepared immediately prior to use by the dentist and formed by mixing DL-2-aminobutyric acid, glycine, sodium hydroxide, sodium chloride, sodium hypochloride, in deionized water.

EP 0 224 599 A1

## CHEMICAL SOLUTION USEFUL IN THE TREATMENT OF TEETH

### FIELD OF THE INVENTION

This invention relates to a chemical solution useful in the treatment of living unextracted teeth by removing caries and/or plaque therefrom while leaving the remainder of the teeth unaffected, e.g., dentin and enamel. In one aspect the invention relates to a caries removal system comprising the chemical solution and a delivery system.

### BACKGROUND OF THE INVENTION

The conventional method for removing carious lesions in teeth is through the use of power operated tools in conjunction with hand manipulated tools. The complete removal of the carious material by the dentist requires the use of rotary cutting instruments as well as hand instruments. Such dental procedures impart a high degree of apprehension and fear to many dental patients, particularly children. Other dental patients have problems and/or reactions with local anesthetic injections which are oftentimes given in those instances where the carious lesions are fairly extensive and deep.

Within the past decade there have been discovered methods which utilize certain chemical solutions to remove dental caries. Such methods substantially reduce and even eliminate the need for mechanical removal of the carious lesion by drills, burrs and hand tools. A suitably designed delivery system for administering the caries removal solution to the carious lesion can consist of a pump, reservoir for the solution, and a handpiece with a uniquely designed handpiece applicator tip. The solution in a fine pulsing stream is delivered through the handpiece to the carious site where it softens the decayed material. The dentist can then remove the softened carious material by light abrasion with the applicator tip while flushing with the solution; note U.S. Pat. No. 3,943,628 issued March 16, 1976.

A preferred chemical solution of choice is one prepared by forming an admixture containing (a) DL-2-aminobutyric acid, (b) sodium hydroxide, (c) sodium chloride, and (d) sodium hypochlorite, in deionized water. The active ingredient is believed to be the N-chlorinated-DL-2-aminobutyric acid and/or the sodium salt thereof. The molar ratios of components (a), (b) and (c) are approximately 1:1:1 and the molar ratio of component (a) relative to component (d) exceeds one, e.g., about 5 to 10, in one liter of deionized water. The resulting pH desirably ranges from about 11-12. The active ingredient presumably reacts with the decalcified, partially degraded collagen of the carious lesion resulting in a softening of the carious material. For further information on this subject reference is made to U.S. Pat. Nos. 3,932,605; 3,991,107; 4,012,842; and 3,776,825.

The aforesaid caries removal solution should be prepared just prior to use in view of its relatively short half life. Though the efficacy of this type of formulation is given a significantly high rating as a caries removal solution, the half-life of the active N-chlorinated ingredient is relatively low. On the other hand, the substitution of glycine for DL-2-aminobutyric acid (all other variables remaining substantially constant) results in a significant increase of the half life of the active N-chlorinated ingredient; however, a significant decrease in efficacy is observed.

### SUMMARY OF THE INVENTION

It is an object of the invention to provide a chemical solution containing N-chlorinated-DL-2-aminobutyric acid or salt thereof useful in the removal of carious material which has a significantly improved half-life while retaining optimum efficacy.

It is another object of the invention to provide an improved caries removal delivery system.

It is a further object of the invention to provide an improved caries removal chemical solution comprising a mixture of two N-chlorinated aminoacids and/or salts thereof which solution is characterized by (a) the high order of efficacy of its most active component and (b) a significantly improved half-life when compared to its most active component.

It is a further object of the invention to provide an improved caries removal chemical solution comprising N-chlorinated-DL-2-aminobutyric acid and N-chlorinated glycine and/or salts thereof which solution (1) is characterized by the relatively high order of efficacy of its most active component, i.e., N-chlorinated-DL-2-aminobutyric acid and/or salts thereof, and (2) is also characterized by an overall significant increase in the half-life when compared to an equivalent caries removal solution which contains N-chlorinated-DL-2-aminobutyric acid and/or salt thereof as the sole aminoacid source.

These and other objects of the invention will become apparent to those skilled in the art from a consideration of this specification.

DETAILED DESCRIPTION OF THE INVENTION

It has now been found quite unexpectedly indeed that the formation of an aqueous solution of DL-aminobutyric acid, glycine, sodium hydroxide, sodium chloride, and sodium hypochloride within a narrow concentration range results in an active caries removal solution which is manifest by improved (longer) half life while retaining the high efficacy characteristic of the active N-chlorinated-DL-2-aminobutyric acid and/or salt thereof. By way of illustration, a (first) solution of N-chloro-DL-2-aminobutyric acid or its Na salt formed by admixing 0.05M DL-2-aminobutyric acid, 0.05M sodium hydroxide, 0.05M sodium chloride, and 0.007M sodium hypochlorite, in sufficient deionized water to make one liter of solution was observed to have a half-life at 20°C of about 63 minutes. A second solution prepared by substituting a mixture of 0.025M DL-2-aminobutyric acid and 0.025M glycine (total of 0.05M of aminoacids) in lieu of the 0.05M DL-2-aminobutyric acid component of the abovesaid first solution resulted in a (second) solution having a half-life at 20°C of about 82 minutes, i.e., a half life increase of approximately 30 percent. One could probably conclude that such increase in half-life was to be expected since N-chlorinated glycine/salt solutions have been observed to exhibit greater half-life values than N-chlorinated-DL-2-aminobutyric acid/salt solutions under comparable conditions. However, the efficacy of such N-chlorinated glycine/salt solution as a caries removal agent is known to be significantly less than the comparable N-chlorinated-DL-2-aminobutyric acid/salt solution. Thus, it was totally unexpected that the (second) solution containing N-chlorinated-DL-2-aminobutyric acid/salt plus N-chlorinated glycine/salt and the (first) solution containing N-chlorinated-DL-2-aminobutyric acid as the sole aminoacid source and at twice the concentration would be characterized by essentially equivalent high orders of efficacy as a caries removal solution.

In the practice of desirable embodiments of the invention, a chemical solution useful in the removal of carious lesions can be prepared by admixing the components listed in Table I:

## TABLE I

| | |
|---|---|
| DL-2-aminobutyric acid | 0.02-0.03 Mol |
| Glycine | 0.02-0.03 Mol |
| Sodium Hydroxide | 0.04-0.06 Mol |
| Sodium Chloride | 0.04-0.06 Mol |
| Sodium Hypochlorite | 0.005-0.01 Mol |
| Deionized Water ([a]) | An amount sufficient to make one liter of solution |
| pH of solution | approx. 10-12 at 20°C |

([a])An amount of deionized water is used to make one liter of chemical solution.

In the practice of a preferred aspect of the invention three caries removal chemical solutions were prepared. The formulations are noted in Table II:

## TABLE II

| COMPONENT | FORMULATION | | |
|---|---|---|---|
| | 1 | 2 | 3 |
| DL-2-amiminobutyric acid | 0.05M | 0.025M | -- |
| Glycine | -- | 0.025M | 0.05M |
| Sodium Hydroxide | 0.05M | 0.05M | 0.05M |
| Sodium Chloride | 0.05M | 0.05M | 0.05M |
| Sodium Hypochlorite | 0.007M | 0.007M | 0.007M |
| Deionized Water (a) | (a) | (a) | (a) |
| Half-Life (at 20°C)(b) | 63 min | 82 min | 108 min |
| pH of Solution(c) | 11.7 | 11.5 | 11.5 |

(a)An amount of deionized water is used to make one liter of chemical solution.

(b)Half life times of aqueous compositions containing the N-chlorinated compound formed in situ.

(c)Determined immediately after forming the chemical solution.

In the illustrative examples, there is employed a caries removal device or system which comprises a nozzle, a source of the chemical solution, pump means preferably connected between said nozzle and said chemical solution under pressure to said nozzle for predetermined intervals preferably separated by dormant intervals. The nozzle can be a tube connected at one end to a handpiece having an applicator tip in the shape of a miniature spoon. In a preferred aspect of the invention the source of the caries removal solution is desirably maintained in two separate reservoirs. One reservoir preferably contains a mixture of the two aminoacids, sodium hydroxide and sodium chloride, in deionized water (Solution A), the other reservoir contains the sodium hypochlorite in deionized water (Solution B). If desired, Solution B could also contain some or all of the sodium chloride and/or sodium hydroxide. Solution A and Solution B are conveniently maintained at normal room temperature, e.g., 20°-21°C., though slightly lower and higher temperatures are, of course, permissible, e.g., 18°-25°C., and upwards to normal body temperature. By adjusting the flow rates and pump action, measured amounts of Solution A and Solution B are withdrawn from the two reservoirs, mixed in a small mixing chamber to form the active caries removal solution, and thereafter applied to the carious site in a fine pulsating stream. A system such as a Water-Pik® can be used, modified with the multichamber reservoirs, mixing and/or heating chamber(s), and handpiece with spoon applicator tip. Active N-chlorinated chemical solution, formed in-situ in the mixing chamber and preferably heated within the chamber to about 37°C., is thereafter applied to the carious site through a tubular shaft connected to and forming a part of the applicator tip in a manner substantially as shown in U.S. Patent No. 3,943,628. Solution A and Solution B may be preheated prior to mixing in the mixing chamber or the active caries removal solution may be heated after exiting from the mixing chamber. Desirably the temperature of the caries removal solution at the time of application to the carious lesion is maintained within a range that affords maximum comfort to the dental patient, e.g., approximately 35° to 38°C.

In the Examples active N-chlorinated chemical solutions (Solutions I and II) are applied to the carious lesion as a pulsating stream through the tubular shaft connected to the applicator tip. The internal diameter of the tip is 0.02 inch. The volume of delivered solution (or flow rate) is 35 5 ml/minute and the temperature of the solution is 37°C. The frequency is desirably maintained at 600-700 cycles/minute. The pressure of the pulsating jet stream varied from 0 to about 50 psi (per cycle of frequency).

Extracted teeth were chosen based on comparable lesion size and consistency of carious material. The teeth used in the Examples were classified as "medium-hard carious material", i.e. (a) resistant to hand probing, (b) not easily removed by mechanical means, and (c) not readily penetrated by dental prober, i.e., a slender hand instrument with a hook-shaped point used by the dentist for examining a cavity.

EXAMPLE 1

| N-CHLORINATED SOLUTION | TIME TO REMOVE CARIOUS MATERIAL |
|---|---|
| I[1] | 5.7 min[4] |
| II[2] | 5.7 min[4] |
| III[3] | >7 min[4] |

(1) Solution I formed by mixing 0.05M DL-2-amino-butyric acid, 0.05M sodium hydroxide, 0.05M sodium chloride, and 0.007 M sodium hypochlorite in sufficient deionized water to make one liter of solution; pH of 11-12.

(2) Solution II formed by mixing 0.025M DL-2-amino-butyric acid, 0.025M glycine, 0.05M sodium hydroxide, 0.05M sodium chloride, and 0.007M sodium hypochlorite in sufficient deionized water to make one liter of solution; pH of 11-12.

(3) Solution III formed by mixing 0.05M glycine, 0.05M sodium hydroxide, 0.05M sodium chloride, 0.007M sodium hypochlorite in sufficient deionized water to make one liter of solution; pH of 11-12.

(4) Average of three classified teeth.

EXAMPLE 2

| N-CHLORINATED SOLUTION | TIME TO REMOVE CARIOUS MATERIAL |
|---|---|
| I[1] | 4 min[4] |
| II[2] | 4 min[4] |
| III[3] | 5.8 min[4] |

(1) See footnote (1), Example 1.

(2) See footnote (2), Example 1.

(3) See footnote (3), Example 1.

(4) Average of 10 classified teeth; after stated time in minutes visual inspection indicates removal of the carious material.

Deionized water, i.e., water purified by passage through an appropriate ion-exchange medium, is preferred since such treatment reduces the quantity of dissolved salts in the water. In turn, the use of deionized water reduces formation of slime which may form over a period of time in the caries removal delivery system.

## Claims

1. A process for the preparation of a chemical solution useful to remove carious material or plaque containing N-chlorinated-aminoacids and/or the salts thereof buffered to a pH value of from 10 to 12 characterized in that said solution is prepared just prior to use by forming a mixture in which the ratio of components are 0.02-0.03M DL-2-aminobutyric acid, 0.02-0.03M glycine, 0.04-0.06M sodium hydroxide, 0.04-0.06M sodium chloride, and 0.005 to 0.01M of sodium hypochlorite, in one liter of water, the resulting solution being characterized by a high order of efficacy of the resulting active N-chlorinated-DL-2-aminobutyric acid or its sodium salt product and a simultaneous significant improvement in the half-life of said N-chlorinated product.

2. The process of claim 1 wherein said solution is formed by admixing 0.025M DL-2-aminobutyric acid, 0.025M glycine, 0.05M sodium hydroxide, 0.05M sodium chloride, and about 0.007M of sodium hypochlorite, in one liter of deionized water, said solution having an initial pH of 11 to 12.

3. A stable two-package kit useful in the treatment of carious lesions or plaque characterized in that the first package contains a mixture of 0.02-0.03M DL-2-aminobutyric acid and 0.02-0.03M glycine and optionally, 0.02-0.03M sodium hydroxide and 0.02-0.03M sodium chloride, and the second package contains 0.005-0.01M sodium hypochlorite and optionally, 0.02-0.03M sodium hydroxide and 0.02-0.03M sodium chloride, with the proviso that the first package and/or second package must contain sodium hydroxide and/or sodium chloride, to form immediately prior to use on the carious lesion or plaque an active chemical solution having a pH of 10 to 12 and containing 0.02-0.03M N-chlorinated-DL-2-aminobutyric acid and/or sodium salt thereof and 0.02-0.03M N-chlorinated glycine and/or sodium salt thereof in one liter of deionized water, said chemical solution being characterized by the high degree of efficacy of N-chlorinated-DL-2-aminobutyric acid or its sodium salt as well as a significant improvement in its overall half-life.

Amended claims in accordance with Rule 86 (2) EPC.

1. A process for the preparation of a chemical solution useful to remove carious material or plaque containing N-chlorinated-aminoacids and/or the salts, characterized in that said solution is prepared just prior to use by forming a mixture in which the ratio of components are 0.02-0.03M DL-2-aminobutyric acid, 0.02-0.03M glycine, 0.04-0.06M sodium hydroxide, 0.04-0.06M sodium chloride, and 0.005 to 0.01M of sodium hypochlorite, in one liter of water, the resulting solution having an initial pH of from 10 to 12 and being characterized by a high order of efficacy of the resulting active N-chlorinated-DL-2-aminobutyric acid or its sodium salt product and a simultaneous significant improvement in the half-life of said N-chlorinated product.

2. The process of claim 1, wherein said solution is formed by admixing 0.025M DL-2-aminobutyric acid, 0.025M glycine, 0.05M sodium hydroxide, 0.05M sodium chloride, and about 0.007M of sodium hypochlorite, in one liter of deionized water, said solution having an initial pH of 11 to 12.

3. A stable two-package kit useful in the treatment of carious lesions or plaque, characterized in that the first package contains a mixture of 0.02-0.03M DL-2-aminobutyric acid and 0.02-0.03M glycine and optionally, 0.02-0.03M sodium hydroxide and 0.02-0.03M sodium chloride, and the second package contains 0.005-0.01M sodium hypochlorite and optionally, 0.02-0.03M sodium hydroxide and 0.02-0.03M sodium chloride, with the proviso that the first package and/or second package must contain sodium hydroxide and/or sodium chloride, to form immediately prior to use on the carious lesion or plaque an active chemical solution having a pH of 10 to 12 and containing 0.02-0.03M N-chlorinated-DL-2-aminobutyric acid and/or sodium salt thereof and 0.02-0.03M N-chlorinated glycine and/or sodium salt thereof in one liter of deionized water, said chemical solution being characterized by the high degree of efficacy of N-chlorinated-DL-2-aminobutyric acid or its sodium salt as well as a significant improvement in its overall half-life.

European Patent
Office

**EUROPEAN SEARCH REPORT**

Application number

— EP 85 11 4149

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-3 998 945 (VIT)<br><br>* Whole document, column 2, lines 25,26; column 4, table *<br><br>----- | | A 61 K 7/22 |

TECHNICAL FIELDS
SEARCHED (Int. Cl.4)

A 61 K

The present search report has been drawn up for all claims

| Place of search | Date of completion of the search | Examiner |
|---|---|---|
| THE HAGUE | 30-06-1986 | BENZ K.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82